# EUROPEAN PATENT APPLICATION

(11) **EP 1 048 278 A2**
(43) Date of publication of application: **02.11.2000**
(21) Application number: 00108754.3
(22) Date of filing: 25.04.2000
(51) Int. Cl.: A61F 13/532, A61F 13/537

(54) **Absorbent structure having differential density**

(30) Priority: 27.04.1999 SE 9901539
(71) Applicant: SCA Hygiene Products AB, 405 03 Göteborg (SE)
(72) Inventor: Berthou, Thomas, 416 51 Göteborg (SE); Fredriksson, Richard, 416 74 Göteborg (SE); Andresen, Anne Guri, 421 43 Västra Frölunda (SE); Österdahl, Eje, 426 58 Västra Frölunda (SE); Utas Hansson, Marie, 437 31 Lindome (SE)
(74) Representative: Egeröd, Lisbeth

(57) **Abstract**

Absorbent structure in an absorbent article such as a sanitary napkin, an incontinence guard, a diaper and the like, said absorbent structure comprises an absorbent body having as seen in its longitudinal direction separate end portions (9,10) and a central mid portion (11) located between said end portions, at which the central mid portions has a higher density and a higher basis weight than the end portions. The central mid portion (11) has substantially the same thickness as the end portions (9,10). The absorbent structure further comprises a liquid acquisition layer (5) with a lower density than the central portion of the absorbent body, said liquid acquisition layer is arranged to cover the central mid portion of the absorbent body but not to extend all the way out the transverse ends (9a,10a) of the absorbent body.

## Description

### Technical field

The present invention refers to an absorbent structure in an absorbent article such as a sanitary napkin, an incontinence guard, a diaper or the like, said absorbent structure comprising an absorbent body having as seen in its longitudinal direction separate end potions and a central mid portion located between said end portions, at which the central mid portions has a higher density and a higher basis weight than the end portions.

### Background of the invention

Many types of absorbent structures in absorbent articles are previously known. Usually they comprise one or more compressed layers of cellulosic fluff pulp, often in combination with so called superabsorbents, which are polymers with the capacity to absorb many times their own weight of water or body fluid.

The body fluid is discharged to the absorbent article in a very limited area, the so called wetting point. It is therefore important that the absorbent article has a high capacity within this area. It is further known that the absorbent body in an absorbent article has different densities in different areas in order to achieve desired spreading patterns in the absorbent body.

Through US-A-4,027,672 it is previously known an absorbent body in the form of a pulp structure where a pattern of different basis weights, e g stripes or circular depressions, have been created. The pulp mat is then compressed to an essentially constant thickness, at which the portions having the higher basis weight gets a higher density as compared to the portions with the lower basis weight. The absorbent body will then get a pattern of portions with different densities.

Through EP-A-0 157 649 there is known an absorbent body with a central mid portion having a higher density and a higher basis weight than the surrounding end portions.

The central mid portion besides has a considerably higher thickness than the surrounding end portions. Through the relatively thick mid portion the absorbent body seems to have a poor fit and pliability against the user's body.

### Object and most important features of the invention

The object of the present invention is to provide an absorbent structure in an absorbent article of the above mentioned kind, which has a high absorbent capacity within the wetting area, which has a low rewet and is soft and pliable against the body.

This has according to the invention been provided by the fact that the central mid portion has substantially the same thickness as the end portions, and that the absorbent structure further comprises a liquid acquisition layer with a lower density than the central portion of the absorbent body, said liquid acquisition layer is arranged to cover the central mid portion of the absorbent body but not to extend all the way out the transverse ends of the absorbent body.

Further characteristics of the invention are disclosed in the description and claims below.

### Description of drawings

The invention will in the following be more closely described with reference to some embodiments shown in the attached drawings.
Fig. 1 shows schematically a view from above of an absorbent article in the form of a sanitary napkin.
Fig. 2 is a section according to the line II-II in Fig.1.
Fig. 3 shows schematically a side view of a pulp core before compression and intended to form an absorbent core according to the invention.
Fig. 4 shows schematically a side view of the pulp core according to Fig. 3 after compression.

### Description of embodiments

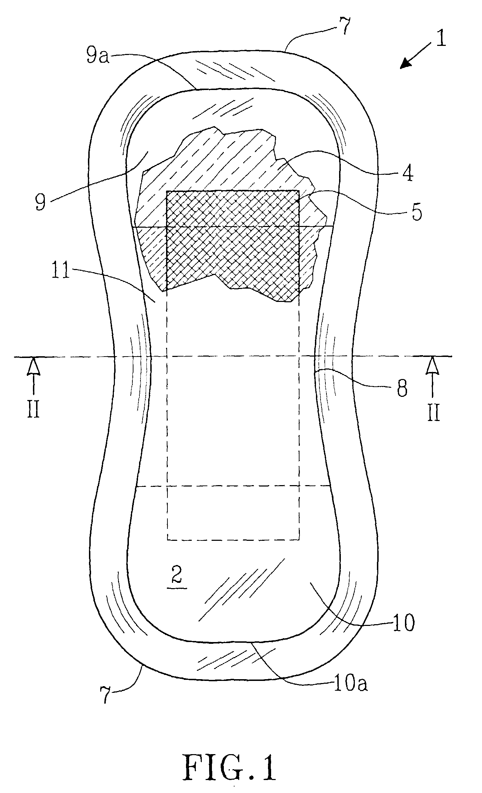

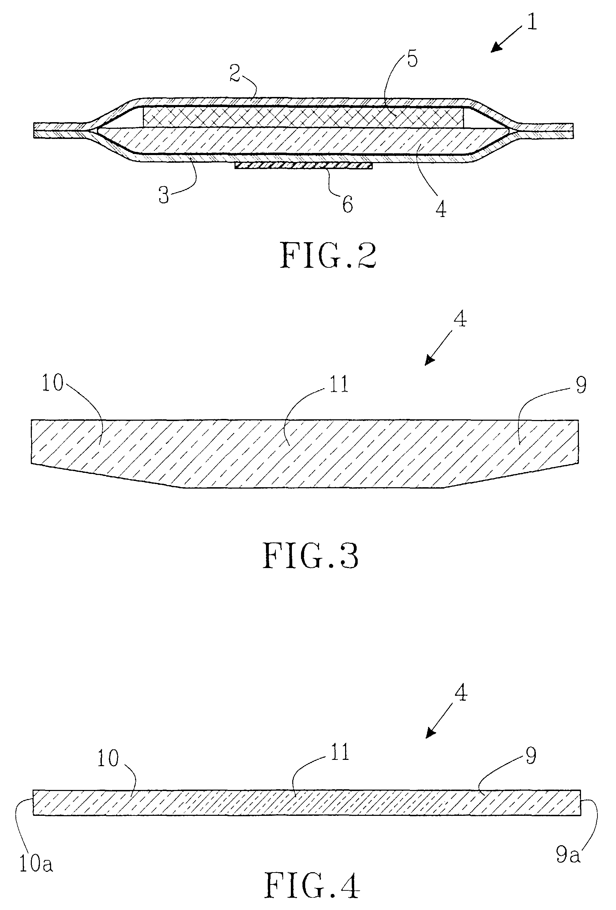

Fig. 1 and 2 shows an embodiment of a sanitary napkin 1 comprising a liquid pervious topsheet 2, a liquid impervious backsheet 3 and an absorbent body 4 enclosed therebetween. On top of the absorbent body 4 there is arranged a liquid acquisition layer 5.

It should be pointed out that the sanitary napkin shown in the drawings is a non-limiting example of an absorbent article. Thus the shape and overall design of the article may vary. The absorbent article may also be an incontinence guard, a diaper or the like. The invention however at first hand refers to a sanitary napkin.

The liquid pervious topsheet 2 may consist of a nonwoven material, for example a spunbond material of synthetic filaments, a meltblown material, a thermobonded material or a bonded carded fibre material. Alternatively it may consist of a perforated plastic film or a perforated laminate of nonwoven and plastic film.

The liquid impervious backsheet 3 may consist of a plastic film, a nonwoven material coated with a liquid barrier material or a hydrophobic nonwoven material which resists liquid penetration. The liquid impervious backsheet 3 may also be a so called breathable material, for example a breathable plastic film or nonwoven, i e a material which is vapour permeable.

On the underside of the liquid impermeable backsheet 3 fastening means in the form of longitudinal stripes 6 of selfadhesive glue are provided. The glue areas are suitably before use covered with a releasable protective strip (not shown) of paper or plastic film treated with release agent. In the embodiment shown the fastening means are longitudinal glue areas. A number of other glue patterns, e g transverse, are of course possible as well as other types of fastening means such as hook and loop type, push buttons, girdles, special underwear or the like.

The sanitary napkin shown in the embodiment is hour glass shaped with broader end portions 7 and a narrower crotch portion 8. The crotch portion 8 is the portion of the sanitary napkin which during use is intended to be applied in the crotch area of the user and serve as a receiving area for the discharged body fluid.

The topsheet 2 and the backsheet 3 have a somewhat larger extension in the plane than the absorbent body 4 and extend outside the edges thereof. The layers 2 and 3 are interconnected within the projecting portions, for example by gluing or welding with ultrasonic or heat.

The absorbent body 4 may be of an optional absorption material, preferably however matformed cellulosic fluff pulp which has been compressed to a desired density. A certain amount of superabsorbent material may if desired be mixed into the fluff pulp.

The absorbent body 4 has as seen in its longitudinal direction separate end portions 9,10 and a central mid portion 11 located between said end portions. The central mid portion 11 has a higher density and a higher basis weight than the end portions 9,10. This difference in density and basis weight is achieved by matforming the core in such a way that there will be more material in the mid portion, which is seen from Fig. 3. The basis weight can vary in different ways, for example as is shown in Fig. 3, by having a substantially even basis weight in the central portion 11, while the basis weight in the end portions gradually decreases out towards the transverse edges 9a, 10a of the core. Alternatively the end portions 9, 10 may have a substantially even basis weight, which however is lower than the basis weight of the mid portion. Finally the mid portion 11 may also have a basis weight that decreases from its central portion.

When this pulp core then is compressed between two rolls so that it becomes essentially evenly thick, it will get different densities in the mid portion and in the ends (Fig. 4).

As seen in its transverse direction the density and the basis weight respectively of the absorbent core 4 within each individual portion 9, 10, 11 may either be substantially constant over the width of the absorbent core or have a lower density / basis weight along the longitudinal edge portions.

A sanitary napkin or other absorbent product which in this way has a higher relative amount of the absorbent material in the wetting area, i e the area receiving the discharged body fluid, will have a higher capacity and better utilization of the absorbent material. The end portions of the sanitary napkin or the like becomes softer due to the lower density and basis weight and feels therefore more comfortable and discreet for the user.

The part of the absorbent core 4 having the higher density, i e the mid portion 11, should constitute between 40 and 70 %, preferably between 50 and 60% of the total length of the absorbent core. In the embodiment shown the uncompressed pulp core has in the mid portion a thickness which is approximately twice as large as the thinnest part of the end portions 9, 10. This involves that the density in the mid portion 11 after compression gets about twice as large as the density at the very end of the end portions 9, 10. Generally the lowest density in the end portions 9, 10 of the absorbent core should amount to between 10 and 75%, preferably between 30 and 60%, of the density of the mid portion 11.

The density in the mid portion 11 should be between 0,1 and 0,9 g/cm³, preferably between 0,1 and 0,4 g/cm³. The density is measured at a pressure of 0,05 N/cm².

On top of the absorbent core 4 there is arranged a liquid acquisition layer 5, e g a wadding of hydrophilic and/or hydrophobic synthetic fibres, regenerated cellulose, such as viscose, and the like. Also other types of porous resilient nonwoven materials, airlaid fibre materials (so called airlaid materials), foam materials and the like can be used as acquisition layers.

The acquisition layer 5 also gives an increased surface dryness of the sanitary napkin and reduces rewet of absorbed liquid against the skin of the user, since it easily lets liquid through to the absorbent core where the liquid then is stored.

Since the absorbent core 4 has a high density zone in the mid portion, which has a higher density than the end portions of the core, the absorbed liquid is inclined to remain in the mid portion 11 and not to the same extent be spread out to the low density areas, i e the end portions 9, 10. This involves that the acquisition layer 5 is of greatest use by covering the mid portion 11 but does not serve any direct purpose by covering the end portions 9, 10. Moreover the ends will be thinner and more pliable. The acquisition layer 5 does therefore as seen in its longitudinal direction only cover the mid portion 11, but for processability reasons it is suitable that the acquisition layer is somewhat longer than the length of the high density zone, i e the mid portion 11, of the absorbent core.

The invention is of course not limited to the embodiment described and shown but can be varied within the scope of the claims.

## Claims

1. Absorbent structure in an absorbent article such as a sanitary napkin, an incontinence guard, a diaper and the like, said absorbent structure comprises an absorbent body having as seen in its longitudinal direction separate end portions (9,10) and a central mid portion (11) located between said end portions, at which the central mid portions has a higher density and a higher basis weight than the end portions,
**characterized in**
that the central mid portion (11) has substantially the same thickness as the end portions (9,10), and that the absorbent structure further comprises a liquid acquisition layer (5) with a lower density than the central portion of the absorbent body, said liquid acquisition layer is arranged to cover the central mid portion of the absorbent body but not to extend all the way out the transverse ends (9a,10a) of the absorbent body.

2. Absorbent structure as claimed in claim 1,
**characterized in**
that the central mid portion (11) constitutes between 40 and 70 %, preferably between 50 and 60% of the total length of the absorbent body (4).

3. Absorbent structure as claimed in claim 1 or 2,
**characterized in**
that the density in the end portions (9,10) of the absorbent core constitutes between 25 and 75%, preferably between 40 and 60%, of the density of the mid portion (11).

4. Absorbent structure as claimed in any of the preceding claims,
**characterized in**
that the central mid portion (11) does not extend all the way out to the longitudinal edges of the absorbent body (4).

5. Absorbent structure as claimed in any of the preceding claims,
**characterized in**
that the material in the absorbent core (4) consists of pulp fibres, possibly in combination with superabsorbent particles.

6. Absorbent structure as claimed in any of the preceding claims,
**characterized in**
that the density in the mid portion (11) is between 0,1 and 0,9 g/cm³, preferably between 0,1 and 0,4 g/cm³.

7. Absorbent article such as a sanitary napkin, an incontinence guard, a diaper or the like and of the kind comprising a liquid pervious topsheet (2), a liquid impervious backsheet (3) and an absorbent structure (4,5) applied therebetween,
**characterized in**
that the absorbent structure (4,5) is of a kind stated in any of claims 1-6.
